# DEMANDE DE BREVET EUROPEEN

(11) **EP 2 803 341 A1**
(43) Date de publication de la demande: **19.11.2014**
(21) Numéro de dépôt: 14305707.3
(22) Date de dépôt: 14.05.2014
(51) Int. Cl.: A61F 5/37

(54) **Echarpe médicale pour l'immobilisation d'un bras d'un patient**

(30) Priorité: 14.05.2013 FR 1354300
(71) Demandeur: Ricordeau, Laurent, 44100 Nantes (FR)
(72) Inventeur: Ricordeau, Laurent, 44100 NANTES (FR); Clarissou, François, 19000 TULLE (FR); Parent, Arthur, 49100 ANGERS (FR); Robin, Florian, 78120 RAMBOUILLET (FR)
(74) Mandataire: Coralis Harle

(57) **Abrégé**

La présente invention concerne une écharpe médicale (2) pour l'immobilisation d'un bras (B) d'un patient (P), comprenant un manchon (5) pour la réception de l'avant-bras (A), et une sangle (6), destinée à être positionnée autour du buste (U) du patient (P).

Cette sangle (6) comprend : - une bande avant (61) en forme d'une anse, destinée à venir reposer sur les épaules (E) du patient (P), et - une bande arrière (62) solidarisée avec ladite bande avant (61) et avec ledit manchon (5), destinée à s'étendre dans le dos du patient (D) et à remonter en regard de la face antérieure abdominale (C) du patient (P).

Une telle structure a notamment l'intérêt d'être particulièrement simple à mettre en place par un patient, cela éventuellement sans l'assistance d'une tierce personne.

## Description

### DOMAINE TECHNIQUE AUQUEL SE RAPPORTE L'INVENTION

La présente invention concerne le domaine du matériel orthopédique. Elle concerne plus particulièrement une écharpe médicale, ou attelle, permettant de traiter les pathologies de l'épaule, du bras (ou membre supérieur) et de la clavicule.

### ARRIERE-PLAN TECHNOLOGIQUE

Le traitement de certaines pathologies de l'épaule, du bras et/ou de la clavicule, nécessite le port prolongé d'une écharpe médicale, par le patient, afin d'immobiliser le bras touché contre son buste.

Cette écharpe médicale comprend un manchon pour la réception de l'avant-bras à immobiliser, en position pliée et sensiblement horizontale.

Ce manchon est classiquement équipé d'une sangle destinée à être positionnée autour du buste du patient.

Or, les sangles des écharpes actuelles s'avèrent en pratique particulièrement complexes à mettre et à enlever, que ce soit pour le patient que pour le personnel soignant.

### OBJET DE L'INVENTION

Dans ce contexte, le demandeur propose une nouvelle écharpe médicale particulièrement ergonomique, pouvant se positionner ou s'enlever sans difficulté et de manière intuitive pour un patient seul.

Sa conception permet également au personnel médical (aide-soignant(e) ou infirmier(e) par exemple) de rapporter cette écharpe sans difficulté sur une personne inconsciente, suite à une opération.

L'écharpe médicale selon l'invention, pour l'immobilisation d'un bras d'un patient dans une position pliée, avec l'avant-bras en position horizontale contre la face antérieure de son abdomen, comprend - un manchon pour la réception dudit avant-bras, et - une sangle, solidarisée avec ledit manchon et destinée à être positionnée autour du buste du patient pour le maintien dudit manchon ; ladite sangle comprend :
- une bande avant, en forme générale d'une anse, munie de deux extrémités terminales qui sont chacune solidarisées avec ledit manchon, cette bande avant étant destinée à venir reposer sur les deux épaules du patient, et
- une bande arrière, dont une extrémité supérieure est solidarisée avec ladite bande avant, et dont une extrémité inférieure est solidarisée avec ledit manchon, ladite bande arrière étant destinée à s'étendre dans le dos du patient et à remonter en regard de la face antérieure abdominale du patient.

Selon d'autres caractéristiques avantageuses, pouvant être prises indépendamment ou en combinaison :
- le manchon de l'écharpe comporte une bordure supérieure destinée à s'étendre au-dessus de l'avant-bras du patient, muni de deux extrémités latérales, et les extrémités terminales de la bande avant sont solidarisées chacune au niveau de l'une des deux extrémités de ladite bordure supérieure ; de préférence, les extrémités terminales de la bande avant sont fixées au manchon par l'intermédiaire de moyens de solidarisation inamovible ;
- l'extrémité supérieure de la bande arrière est solidarisée en un point milieu de la bande avant, situé au milieu de sa longueur ou au moins approximativement au milieu de sa longueur.
- l'extrémité inférieure de la bande arrière est solidarisée avec le manchon par le biais de moyens de solidarisation amovible ; de préférence, la face avant du manchon, destinée à être orientée à l'opposé du patient, comporte au moins une bande auto-agrippante, et l'extrémité inférieure de la bande arrière comporte une bande auto-agrippante complémentaire ; le manchon est de préférence encore délimité par deux bordures latérales, et la bande auto-agrippante du manchon s'étend sur toute la longueur, ou au moins approximativement toute la longueur, de la face avant du manchon, séparant ses deux bordures latérales ;
- le manchon se présente sous la forme d'une pièce repliée qui est délimitée par deux bordures latérales, correspondant aux bordures latérales du manchon, et par deux bordures longitudinales ; et les bordures longitudinales sont solidarisées ensemble par le biais de moyens de solidarisation amovible ; de préférence, ces moyens de solidarisation amovible comportent deux bandes auto-agrippantes, ménagées chacune le long de l'une des deux bordures longitudinales du manchon.

La présente invention concerne également un équipement médical pour l'immobilisation du bras d'un patient dans une position pliée, avec l'avant-bras en position horizontale contre la face antérieure de son abdomen, lequel équipement comprend :
- une écharpe médicale telle que définie ci-dessus, et
- une bande horizontale de contention, dite encore « ceinture thoracique » dont l'une des extrémités est équipée d'une boucle de serrage et dont l'autre des extrémités forme un tronçon de bande coopérant avec ladite boucle de serrage pour définir un brin de traction libre s'étendant depuis ladite boucle de serrage ; cette boucle de serrage est structurée de sorte que ledit tronçon de bande est, d'une part, mobile en translation au sein de ladite boucle de serrage lorsqu'une traction est exercée sur ledit brin de traction libre pour provoquer une réduction de la longueur de ladite bande de contention et, d'autre part, verrouillable en translation par rapport à ladite boucle de serrage pour le maintien de la longueur de la bande de contention une fois ajustée.

La présente invention concerne également cette bande horizontale de contention en tant que telle.

### DESCRIPTION DETAILLEE DE L'INVENTION

La présente invention sera encore illustrée, sans être aucunement limitée, par la description suivante d'une forme de réalisation particulière d'un équipement médical, en relation avec les dessins annexés dans lesquels :
- la figure 1 est une vue générale de l'équipement médical selon l'invention, à savoir une écharpe médicale et une bande horizontale de contention, porté par un patient qui est vu du côté de sa face antérieure et dont seul le buste est illustré ;
- la figure 2 représente encore l'équipement médical en situation sur le buste du patient selon la figure 1, vu ici du côté de sa face postérieure ;
- la figure 3 est une vue isolée de l'écharpe médicale portée par le patient sur les figures 1 et 2 ;
- la figure 4 représente, également de manière isolée, la bande horizontale de contention portée par le patient sur les figures 1 et 2 ;
- la figure 5 représente, en détails et de manière agrandie, la boucle de serrage équipant la bande horizontale de contention selon la figure 4.

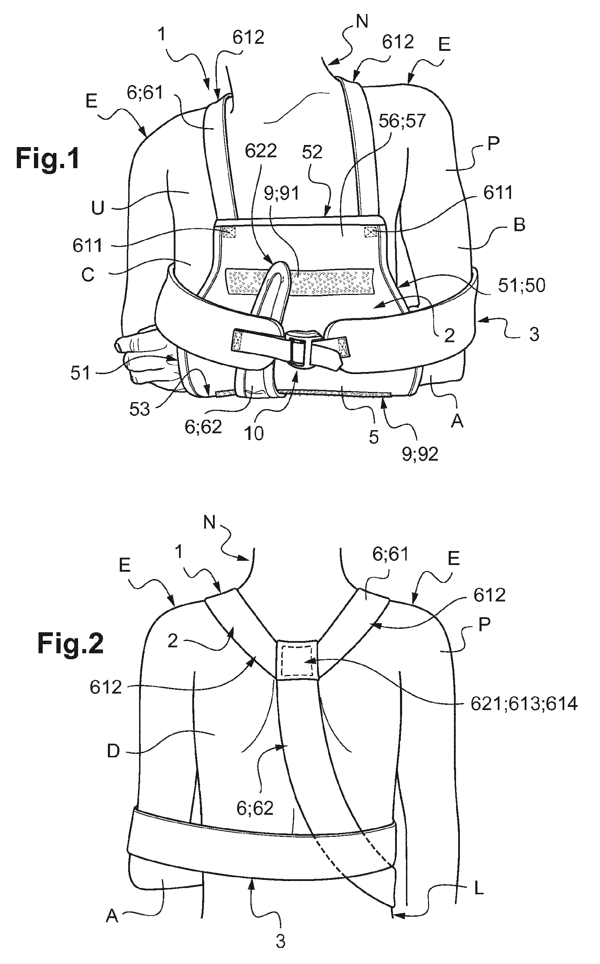

L'équipement médical 1, porté par un individu P dont seul le buste U est ici représenté sur les figures 1 et 2, est destiné aux patients ayant subis un traumatisme de l'épaule, de la clavicule et/ou du bras.

Cet équipement médical 1 est en pratique prévu pour immobiliser le bras atteint/invalide B du patient P dans une position pliée, avec l'avant-bras A en position horizontale contre la face antérieure de son abdomen C.

Le terme « bras » désigne ici le membre supérieur du patient P, dans sa totalité. Cet équipement médical 1 comprend :
- une écharpe médicale 2, pour la réception et le maintien de l'avant-bras A dans la position pliée et sensiblement horizontale, et
- une bande horizontale de contention 3, dite encore « bande thoracique », pour ceinturer le coude du bras invalide B et l'écharpe médicale 2, afin de compléter l'immobilisation recherchée.

L'écharpe médicale 2, illustrée sur les figures 1 à 3, comprend :
- un manchon 5, pour la réception de l'avant-bras A, et
- une sangle 6, solidarisée avec le manchon 5 et destinée à être positionnée autour du buste U du patient P pour le maintien en position de ce manchon 5.

Le manchon 5, illustré en particulier sur la figure 1, consiste en un organe tubulaire allongé qui est réalisé dans un matériau approprié, en particulier un matériau textile.

Ce manchon 5 est muni d'un logement longitudinal traversant 50 pour le passage de l'avant-bras A du patient P (figure 1).

Ce manchon 5 est délimité par deux bordures latérales 51 qui définissent les deux ouvertures opposées de ce logement longitudinal traversant 50. Ces bordures latérales 51 sont ainsi destinées à s'étendre au niveau du poignet et du coude de cet avant-bras A immobilisé.

Le manchon 5 est délimité également par une bordure longitudinale supérieure 52 et par une bordure longitudinale inférieure 53, destinées à s'étendre respectivement au-dessus et au-dessous de l'avant-bras A rapporté dans le manchon 5 (figure 1).

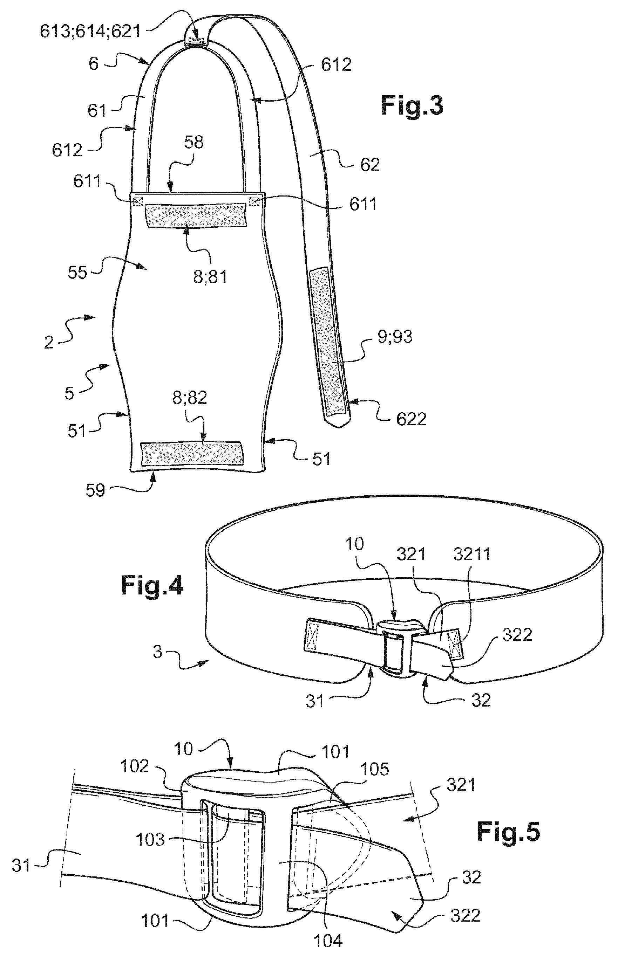

Ce manchon 5 présente encore deux surfaces opposées :
- une surface intérieure 55 (figure 3), délimitant le logement longitudinal traversant 50, et
- une surface extérieure 56 (figure 1), orientée vers l'extérieur.

Cette surface extérieure 56 est divisée en deux faces opposées, s'étendant entre les deux bordures longitudinales 52, 53 précitées, à savoir - une face arrière (non visible ici) destinée à venir appuyer sur la face antérieure de l'abdomen C du patient P, et - une face avant 57, destinée à s'étendre à l'opposé du patient.

Tel qu'illustré sur la figure 3, le manchon 5 consiste ici en une pièce de forme générale rectangulaire, qui est destinée à être repliée en deux.

Le manchon 5, en configuration ouverte (figure 3), est délimité - par deux bordures latérales, correspondant aux bordures latérales 51 précitées, et - par deux bordures longitudinales opposées 58, 59, destinées à former ensemble la bordure longitudinale supérieure 52 précitée.

Une première bordure longitudinale 58 de ce manchon 5 ouvert est solidarisée avec la sangle 6 ; une seconde bordure longitudinale 59 de ce manchon 5 ouvert est libre.

Les deux bordures longitudinales 58, 59 de ce manchon 5 ouvert sont solidarisées ensemble par le biais de moyens de solidarisation amovible 8, pour former ensemble la bordure supérieure 52 du manchon 5.

Ces moyens de solidarisation amovible 8 consistent ici en deux bandes auto-agrippantes 81, 82 complémentaires.

Elles sont ménagées sur la surface intérieure 55 du manchon 5, et chacune le long de l'une des deux bordures longitudinales 58, 59 de ce manchon 5 ouvert.

Un tel manchon 5 « ouvrable/refermable » a en particulier pour intérêt de permettre au patient de déplier son avant-bras A, cela sans retirer son écharpe médicale 2, ce qui est utile notamment pour sa rééducation.

De son côté, la sangle 6 de cette écharpe médicale 2 selon l'invention est destinée à faciliter la mise en place de cette dernière par le patient P, cela sans l'assistance d'une tierce personne et d'une manière particulièrement intuitive.

Tel que représenté sur les figures 1 à 3, cette sangle 6, en forme générale de Y, se compose ici de deux bandes associées :
- une bande dite « avant » 61 rappelant une sorte d'anse, recourbée en forme de U ou de V, solidarisée avec le manchon 5 et destinée à venir reposer sur les épaules E du patient P, et éventuellement au niveau ou en-dessous de sa nuque N, après passage derrière la tête, et
- une bande dite « arrière » 62, visible sur les figures 2 et 3, s'étendant depuis ladite bande avant 61 de sorte à s'étendre dans le dos D du patient P (figure 2) et à remonter en regard de sa face abdominale antérieure C (figure 1).

Les bandes avant 61 et arrière 62 consistent chacune en une bande de matériau approprié, en particulier un matériau textile, de forme rectangulaire allongée.

La longueur de la bande avant 61 est adaptée de sorte que le manchon 5 soit suspendu en regard de l'abdomen C du patient P, lorsque ce dernier enfile cette bande avant 61 autour de sa nuque N.

La bande avant 61 comporte deux extrémités terminales 611 qui sont chacune solidarisées avec le manchon 5, avantageusement au niveau de sa bordure supérieure 52. De préférence, chaque extrémité terminale 611 est solidarisée au niveau de l'une des deux extrémités de la bordure supérieure 52 du manchon 5 (en particulier au niveau de la première bordure longitudinale 58 de ce manchon 5) et à proximité de l'une de ses deux bordures latérales 51, cela sur la face arrière de ce manchon 5.

Cette solidarisation est ici réalisée par le biais de moyens de fixation inamovible, tels qu'une couture, un collage ou un thermosoudage.

En l'occurrence, cette bande avant 61 est constituée de deux tronçons de bande 612 identiques, dont - une extrémité inférieure 611 correspond à l'une des deux extrémités terminales 611 de la bande avant 61 et - une extrémité supérieure 613 est solidarisée avec l'extrémité supérieure 613 de l'autre tronçon de bande 612, formant ainsi le point milieu 614 de cette bande avant 61.

Cette bande avant 61, de manière alternative, pourrait être constituée par un tronçon de bande unique, formant une partie saillante recourbée.

La bande arrière 62 comporte également deux extrémités terminales :
- une extrémité supérieure 621, assemblée avec la bande avant 61 précitée (figure 2), et
- une extrémité inférieure 622, assemblée avec le manchon 5 (figure 1).

L'extrémité supérieure 621 de cette bande arrière 62 est solidarisée en un point milieu de la bande avant 61, c'est-à-dire un point situé au milieu de la longueur de cette bande avant 61, ou au moins approximativement au milieu de cette longueur (à égale distance des deux extrémités terminales 611 de cette bande avant 61). En l'espèce, cette extrémité supérieure 621 de la bande arrière 62 est solidarisée avec l'extrémité supérieure 613 de chacun des deux tronçons de bande 612 formant la bande avant 61.

Cette extrémité supérieure 621 de la bande arrière 62 est ici solidarisée avec la bande avant 61 par le biais de moyens de solidarisation inamovible, par exemple de type couture, collage ou thermosoudage.

Tel que développé ci-après, cet agencement de la bande arrière 62 permet, à cette dernière, de s'étendre le long de la colonne vertébrale du patient P lorsqu'il enfile la bande avant 61 autour de sa nuque N.

Le patient P peut alors facilement saisir cette bande arrière 62 dans son dos D avec son bras libre, que ce soit le droit ou le gauche, en vue de sa manipulation.

L'extrémité inférieure 622 de cette bande arrière 62 est quant à elle solidarisée avec le manchon 5 par le biais de moyens de solidarisation amovible 9, en l'occurrence de type bandes auto-agrippantes.

Pour cela, la face avant 57 et la bordure inférieure 53 du manchon 5 comportent chacune une bande auto-agrippante 91, 92 (figure 1) ; et l'extrémité inférieure 622 de la bande arrière 62 comporte une bande auto-agrippante complémentaire 93 (figure 3) sur sa face destinée à venir en regard de la face avant 57 et la bordure inférieure 53 du manchon 5.

Chaque bande auto-agrippante 91, 92 du manchon 5 consiste en une bande rectangulaire qui s'étend sur toute la longueur, ou au moins approximativement toute la longueur, de ce manchon 5. En d'autres termes, chaque bande auto-agrippante 91, 92 s'étend entre les deux bordures latérales 51 précitées du manchon 5.

Cette caractéristique permet un positionnement optimal de l'extrémité inférieure 622 de la bande arrière 62, quel que soit l'avant-bras A (droit ou gauche) glissé dans le manchon 5.

La bande horizontale de contention 3 consiste également en une bande réalisée en un matériau approprié, en particulier un matériau textile.

Cette bande horizontale de contention 3 se compose d'une pièce de forme générale rectangulaire allongée, qui se termine par deux sangles :
- une première sangle 31, équipée d'une boucle de serrage 10, et
- une seconde sangle 32, destinée à coopérer avec cette boucle de serrage 10.

La seconde sangle 32 est mobile en translation au sein de cette boucle de serrage 10, ce qui permet l'ajustement en longueur de la bande horizontale de contention 3.

La boucle de serrage 10 est du type de celles équipant notamment les sangles de sacs à dos, pour leur ajustement en longueur.

Cette boucle de serrage 10 se présente ici sous la forme d'une pièce plastique monobloc comprenant deux longerons 101 reliés par quatre traverses (figure 5), à savoir :
- une première traverse 102 autour de laquelle est enroulée la première sangle 31 de la bande horizontale de contention 3, pour la solidarisation inamovible de la boucle de serrage 10 sur cette bande horizontale de contention 3, et
- une succession de trois traverses 103, 104 et 105, autour desquelles est convenablement agencée la sangle mobile 32 de la bande horizontale de contention 3.

Partant de la première traverse 102, les différentes traverses 103, 104 et 105 sont désignées, respectivement, sous le nom de « traverse amont 103 », « traverse intermédiaire 104 » et « traverse aval 105 ».

En particulier, la sangle mobile 32 comporte :
- un brin aller 321 dont une extrémité 3211 est attachée à la bande horizontale de contention 3 (figure 4), et qui passe au-dessus de la traverse aval 105 et au-dessous des traverses intermédiaire 104 et amont 103, et
- un brin retour 322, passant au-dessous de la traverse intermédiaire 104 tout en passant au-dessus des traverses amont 103 et aval 105.

Ce brin retour 322 est ainsi pincé notamment entre le brin aller 321 et la traverse intermédiaire 104. Il forme un brin de traction libre, sortant de la boucle de serrage 10.

La structure de cette boucle de serrage 10, classique en soi, permet ainsi une manoeuvre en translation de la sangle mobile 32, dans deux sens, au sein de la boucle de serrage 10.

Lorsqu'une traction est exercée par un individu sur le brin retour 322, le brin aller 321 est raccourci avec un allongement proportionnel de ce brin retour 322. On obtient ainsi une réduction de la longueur de la bande de contention 3.

Cette boucle de serrage 10 assure également un verrouillage automatique, en translation, de la sangle mobile 32 pour le maintien de la longueur de la bande de contention 3 une fois ajustée.

Il est également possible de manoeuvrer en sens inverse la sangle mobile 32 par rapport à la boucle de serrage 10, de sorte que le brin retour 322 soit raccourci, avec un allongement proportionnel du brin aller 321. On obtient ainsi un allongement de la longueur de la bande de contention 3.

En pratique, le patient P devant immobiliser l'un de ses avant-bras A enfile tout d'abord l'écharpe médicale 2.

Pour cela, il peut, s'il le souhaite, manoeuvrer le manchon 5 dans sa configuration ouverte (avec les bandes auto-agrippantes complémentaires 81, 82 désolidarisées l'une de l'autre).

Le patient P passe ensuite la bande avant 61 de la sangle 6 autour de sa nuque N et dépose cette bande avant 61 sur ses deux épaules E (figure 2), tout en positionnant le manchon 5 sur la face antérieure de son abdomen C (figure 1).

La bande avant 61 s'étend ainsi, successivement depuis le manchon 5, contre le torse du patient P, sur ses épaules E et dans son dos D. Les tronçons de bande 612 de cette bande avant 61 se rejoignent ici dans le dos D du patient P.

Le manchon 5 est ainsi suspendu autour de sa nuque N, par l'intermédiaire de sa bande avant 61.

Le patient P positionne ensuite son avant-bras A contre la surface intérieure 55 du manchon 5 en position ouverte, puis replie et ferme ce manchon 5 autour de son avant-bras A de sorte à mettre en oeuvre les moyens de solidarisation amovible 8.

Le patient P saisit la bande arrière 62 de la sangle 6, cette dernière se situant approximativement le long de sa colonne vertébrale, cela de manière relativement aisée au moyen de son bras libre.

Il ramène l'extrémité libre 622 de cette bande arrière 62 vers la face antérieure de son abdomen C, tout en exerçant une légère traction sur cette extrémité libre 622 ; il la positionne contre le manchon 5, en particulier sous sa bordure longitudinale inférieure 53 et contre sa face avant 57, de sorte à mettre en oeuvre les moyens de solidarisation amovible 9.

Le patient P positionne de préférence le point milieu 614 de la bande avant 61, au moins approximativement, au niveau de sa colonne vertébrale, avantageusement en-dessous de sa nuque et entre ses omoplates (figure 2) ; cette configuration vise à obtenir une répartition optimale de l'appui de la sangle 6 sur le buste du patient P.

L'orientation transversale de chaque bande auto-agrippante 91, 92 équipant le manchon 5 permet au patient P de positionner, de manière ambidextre, l'extrémité inférieure 622 de la bande arrière 62.

La bande arrière 62 s'étend ainsi, successivement et partant de la bande avant 61, dans le dos D du patient P, contre la face abdominale latérale L (figure 2) et en regard de la face abdominale antérieure C (figure 1).

Le patient P peut ensuite mettre en place la bande horizontale de contention 3.

Pour cela, dans un premier temps, cette bande de contention 3 est avantageusement rallongée, de sorte à faciliter son positionnement.

Lorsque cette bande horizontale de contention 3 est convenablement agencée au-dessus de son bras B et du manchon 5 en position, il suffit au patient P d'exercer une traction sur le brin de traction libre 322 afin d'ajuster convenablement sa longueur.

Si le patient P souhaite mouvoir son avant-bras A, il lui suffit de desserrer la bande horizontale de contention 3 et de déplier le manchon 5 en désactivant ses moyens de solidarisation amovible 8.

De manière générale, cet équipement médical a l'intérêt d'être particulièrement ergonomique, notamment lors de sa mise en place par un patient seul.

Encore de manière générale, la sangle de l'écharpe médicale selon l'invention, pourrait être adaptée avec un manchon ne présentant pas la fonctionnalité d'ouverture ; les bordures longitudinales 58, 59 de ce manchon 5 sont avantageusement solidarisées ensemble par le biais de moyens de solidarisation inamovible, tels qu'une couture, un collage ou un thermosoudage.

De manière réciproque, le manchon ouvrant, décrit ici, pourrait être adapté avec toute autre forme de sangle appropriée.

## Revendications

1. Echarpe médicale pour l'immobilisation d'un bras (B) d'un patient (P) dans une position pliée, avec l'avant-bras (A) en position horizontale contre la face antérieure de son abdomen (C), laquelle écharpe médicale (2) comprend - un manchon (5) pour la réception dudit avant-bras (A), et - une sangle (6), solidarisée avec ledit manchon (5) et destinée à être positionnée autour du buste (U) du patient (P) pour le maintien dudit manchon (5),
**caractérisée en ce que** ladite sangle (6) comprend :
- une bande avant (61) en forme générale d'une anse, munie de deux extrémités terminales (611) qui sont chacune solidarisées avec ledit manchon (5), ladite bande avant (61) étant destinée à venir reposer sur les deux épaules (E) du patient (P), et
- une bande arrière (62) dont une extrémité supérieure (621) est solidarisée avec ladite bande avant (61) et dont une extrémité inférieure (622) est solidarisée avec ledit manchon (5), ladite bande arrière (62) étant destinée à s'étendre dans le dos du patient (D) et à remonter en regard de la face antérieure abdominale (C) du patient (P).

2. Echarpe médicale selon la revendication 1, **caractérisée en ce que** le manchon (5) comporte une bordure supérieure (52) destinée à s'étendre au-dessus dudit avant-bras (A), et **en ce que** les extrémités terminales (611) de la bande avant (61) sont solidarisées chacune au niveau de l'une des deux extrémités de ladite bordure supérieure (52).

3. Echarpe médicale selon la revendication 2, **caractérisée en ce que** les extrémités terminales (611) de la bande avant (61) sont fixées au manchon (5) par l'intermédiaire de moyens de solidarisation inamovible.

4. Echarpe médicale selon l'une quelconque des revendications 1 à 3, **caractérisée en ce que** l'extrémité supérieure (621) de la bande arrière (62) est solidarisée en un point milieu (614) de la bande avant (61), situé au milieu de sa longueur, ou au moins approximativement au milieu de sa longueur.

5. Echarpe médicale selon l'une quelconque des revendications 1 à 4, **caractérisée en ce que** l'extrémité inférieure (622) de la bande arrière (62) est solidarisée avec le manchon (5) par le biais de moyens de solidarisation amovible (9).

6. Echarpe médicale selon la revendication 5, **caractérisée en ce que** la face avant (57) du manchon (5), destinée à être orientée à l'opposé du patient (P), comporte au moins une bande auto-agrippante (91, 92), et **en ce que** l'extrémité inférieure (622) de la bande arrière (62) comporte une bande auto-agrippante (93) complémentaire.

7. Echarpe médicale selon la revendication 6, **caractérisée en ce que** le manchon (5) est délimité par deux bordures latérales (51), et **en ce que** la bande auto-agrippante (91, 92) du manchon (5) s'étend sur toute la longueur, ou au moins approximativement toute la longueur, de la face avant (57) du manchon (5), séparant ses deux bordures latérales (51).

8. Echarpe médicale selon l'une quelconque des revendications 1 à 7, **caractérisée en ce que** le manchon (5) se présente sous la forme d'une pièce repliée qui est délimitée par deux bordures latérales (51), correspondant aux bordures latérales (51) du manchon (5), et par deux bordures longitudinales (58, 59), et **en ce que** les bordures longitudinales (58, 59) sont solidarisées ensemble par le biais de moyens de solidarisation amovible (8).

9. Echarpe médicale selon la revendication 8, **caractérisée en ce que** les moyens de solidarisation amovible (8) comportent deux bandes auto-agrippantes (81, 82), ménagées chacune le long de l'une des deux bordures longitudinales (58, 59) du manchon (5).

10. Equipement médical pour l'immobilisation du bras (B) d'un patient (P) dans une position pliée, avec l'avant-bras (A) en position horizontale contre la face antérieure de son abdomen (C), ledit équipement (1) comprenant :
- une écharpe médicale (2) selon l'une quelconque des revendications 1 à 9, et
- une bande horizontale de contention (3), dont l'une des extrémités (31) est équipée d'une boucle de serrage (10) et dont l'autre des extrémités (32) forme un tronçon de bande mobile coopérant avec ladite boucle de serrage (10) pour définir un brin de traction libre (322) s'étendant depuis ladite boucle de serrage (10), laquelle boucle de serrage (10) est structurée de sorte que ledit tronçon de bande mobile (32) est, d'une part, mobile en translation au sein de ladite boucle de serrage (10) lorsqu'une traction est exercée sur ledit brin de traction libre (322) pour provoquer une réduction de la longueur de ladite bande de contention (3) et, d'autre part, verrouillable en translation par rapport à ladite boucle de serrage (10) pour le maintien de la longueur de la bande de contention (3) une fois ajustée.
